# EUROPEAN PATENT SPECIFICATION

(11) **EP 2 285 219 B1**
(45) Date of publication and mention of the grant of the patent: **18.07.2018**
(21) Application number: 09743054.0
(22) Date of filing: 07.05.2009
(51) Int. Cl.: A01N 43/62, A61K 31/55, A61K 9/16, A61K 9/20, A61K 9/50

(54) **CONTROLLED RELEASE FORMULATIONS OF ALPRAZOLAM**
ALPRAZOLAMFORMULIERUNGEN MIT GESTEUERTER FREISETZUNG
FORMULATIONS À LIBÉRATION CONTRÔLÉE D ALPRAZOLAM

(30) Priority: 08.05.2008 US 51522 P
(43) Date of publication of application: 23.02.2011
(73) Proprietor: Supernus Pharmaceuticals, Inc., Rockville, MD 20850 (US)
(72) Inventor: VIEIRA, Michael, L., Gaithersburg Maryland 20882 (US); BHATT, Padmanabh, P., Rockville Maryland 20850 (US)
(74) Representative: Walcher, Armin
(86) International application number: PCT/US2009/002820
(87) International publication number: WO 2009/137067

(56) References cited:
- CA-A1- 2 635 313
- US-A1- 2005 163 843
- US-A1- 2006 204 578
- US-A1- 2006 217 367

## Description

### CROSS-REFERENCE TO RELATED PATENT APPLICATIONS

This application claims priority from Provisional Application US Application 61/051,522, filed 05/08/2008.

### BACKGROUND OF THE INVENTION

Alprazolam is an antianxiety agent of the benzodiazepine class. The chemical name of alprazolam is 8-chloro-1-methyl-6-phenyl-4H-s-triazolo[4,3-alpha][1,4]benzodiazepine. Used primarily for short-term relief of mild to moderate anxiety and nervous tension, alprazolam is also effective in the treatment of activity depression or panic attacks.

Alprazolam is available as an immediate release oral tablet (e.g., XANAX®) as well as an extended release tablet (e.g., XANAX XR®). XANAX immediate release tablets contain 0.25mg, 0.5mg, 1mg or 2mg of alprazolam and are indicated for the management of anxiety disorder and for the treatment of panic disorder (with or without agoraphobia). XANAX XR extended release tablets contain 0.5mg, 1mg, 2mg or 3mg of alprazolam and are indicated for the treatment of panic disorder (with or without agoraphobia). XANAX XR is a hypromellose-based extended release tablet.

The immediate release product produces efficacious levels of drug in the systemic circulation, but has to be administered multiple times a day (e.g., up to three to four times daily) in order to maintain efficacy. The extended release tablet, dosed once daily, produces a sustained drug plasma concentration profile with a relative bioavailability of 100% as compared to the XANAX immediate release tablet. Although having 100% relative bioavailability, the XANAX XR product has two shortcomings in that 1) the duration of effect is reported by patients to be not long enough and thus the medication needs to be dosed twice daily in certain patient populations and 2) patients report that there is a lack of effectiveness of the product immediately upon administration.

Thus, there remains a need for an alprazolam formulation that would address the shortfalls of the current XANAX XR product. Additionally, there is a need for an alprazolam formulation that has the potential to better control the fluctuations in drug plasma concentration at steady state (i.e., the difference between the peak to trough values) by delivering the drug using a combination of release rates. Better control of the drug plasma concentration fluctuations can result in lower adverse events as compared to the currently available immediate release and extended release alprazolam drug therapies.

US 2006/0204578A1 discloses a dual controlled release osmotic device. CA 2 635 313 discloses a multi-layered tablet with triple release combination.

### BRIEF SUMMARY OF THE INVENTION

The current invention provides a controlled release formulation of alprazolam for a once a day oral administration pursuant to claim 1 that is effective immediately upon administration, as well as through a period of time of at least 24 hours after administration. This once a day formulation of alprazolam comprises at least one immediate release (IR) component, and at least one extended release (XR) component comprising a release controlling material. The IR component of the formulation is such that at least 90% of the active agent contained in it is released within 30 min of administration. The XR component of the novel formulation releases alprazolam in vivo in a sustained manner, and may be customized to fit a specific pre-determined release profile. In one embodiment, 80% of the total amount of alprazolam is released in 10 to 24 hours.

The current invention additionally provides dosage forms for once a day administration of the controlled release formulation, as well as the method of treatment of generalized anxiety disorder, panic disorder, and anxiety associated with other CNS disorders using this formulation.

### BRIEF DESCRIPTION OF THE DRAWINGS

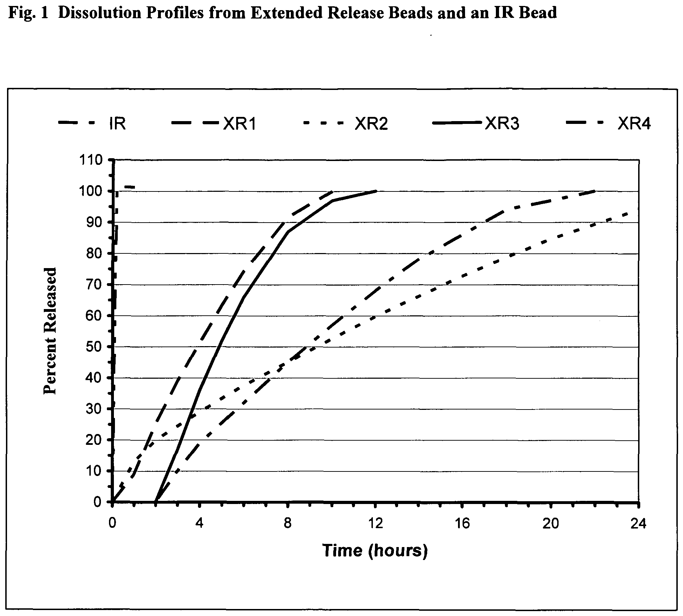

**Fig. 1** presents dissolution profiles of the extended release beads XR1, XR2, XR3, XR4, and an immediate release IR bead.

### DEFINITIONS

For the purposes of this invention, the term "alprazolam" includes alprazolam or any pharmaceutically acceptable salt thereof, as well as any crystalline and non-crystalline form, and any polymorph(s).

An "immediate release formulation" refers to a formulation that releases greater than or equal to about 80% of the pharmaceutical agent in less than or equal to about 1 hour.

"Sustained release" or "extended release" or "controlled release" is defined herein as release of a pharmaceutical agent in a continuous manner over a prolonged period of time.

By "prolonged period of time" is meant a continuous period of time of greater than about 1 hour, preferably, greater than about 4 hours, more preferably, greater than about 8 hours, more preferably greater than about 12 hours, more preferably still, greater than about 16 hours up to more than about 24 hours.

As used herein, unless otherwise noted, "rate of release" or "release rate" of a drug refers to the quantity of drug released from a dosage form per unit time, e.g., milligrams of drug released per hour (mg/hr) or a percentage of the total drug dose released per hour. Drug release rates for dosage forms are typically measured in vitro, i.e., a quantity of drug released from the dosage form per unit time measured in a laboratory under appropriate conditions and in a suitable fluid. The time at which a specified percentage of the drug within a dosage form has been released from said dosage form is referred to as the "Tx" value, where "x" is the percent of drug that has been released.

The release rates referred to herein are determined by placing the dosage form to be tested in an appropriate dissolution media bath. Aliquots of the medium, collected at pre-set intervals, are then injected into a chromatographic system fitted with an appropriate detector to quantify the amounts of drug released during the testing intervals.

As used herein, unless otherwise noted, "delayed release" means there is no release of the pharmaceutical agent until a point in time where release begins.

"C" denotes the concentration of the drug in blood plasma, or serum, of a subject, and is generally expressed as mass per unit volume, for example nanograms per milliliter (ng/ml). For convenience, this concentration may be referred to herein as "drug plasma concentration," "plasma drug concentration" or "plasma concentration" which is intended to be inclusive of a drug concentration measured in any appropriate body fluid or tissue. The plasma drug concentration at any time following drug administration is referenced as Cₜᵢₘₑ, as in C₄ₕᵣ or C₉ₕᵣ, etc.

The maximum plasma drug concentration during the dosing period is referenced as Cₘₐₓ, while Cₘᵢₙ refers to the minimum plasma drug concentration during the dosing interval; and Cₐᵥₑ refers to an average concentration during the dosing interval.

The "degree of fluctuation" for a dosing period is defined as a quotient (Cₘₐₓ - Cₘᵢₙ)/Cₐᵥₑ.

Persons skilled in the art will appreciate that plasma drug concentrations obtained in individual subjects will vary due to inter-patient variability in the many parameters affecting drug absorption, distribution, metabolism and excretion. For this reason, unless otherwise indicated, when a plasma drug concentration is listed, the value listed is the calculated mean value based on values obtained from a group of subjects tested.

The term "bioavailability" refers to an extent to which, and sometimes rate at which, the active moiety (drug or metabolite) enters the systemic circulation, thereby gaining access to the site of action.

"AUC" is the area under the plasma drug concentration-time curve and is considered to be the most reliable measure of bioavailability. The AUC is directly proportional to the total amount of unchanged drug that reaches the systemic circulation.

Side effect is defined herein as any secondary, usually adverse, effect of a drug.

The term "beads," as used herein, includes, without any limitations on the nature and size thereof, any particles, spheres, beads, granules, pellets, particulates or any structural units that may be incorporated into an oral dosage form.

For the purposes of this application, two formulations are given in the "equivalent amount" if they produce an AUC within 80% to 125% of each other for the same period of time.

Throughout this application, "administered tid" means that 1/3 of the total daily dose of the active agent is being administered every 8 hours.

### DETAILED DESCRIPTION OF THE INVENTION

The current invention provides a controlled release formulation of alprazolam for once a day administration to a mammalian subject according to claim 1, which formulation releases alprazolam along a pre-determined release profile. In one embodiment, the profile is such that the blood concentration of the active agent at 24 hours after a single dose administration is not less than 75% of C_{24IR}, wherein C_{24IR} refers to the plasma concentration of drug obtained from the equivalent amount of alprazolam administered as an immediate release formulation TID.

Alternatively, the pre-determined release profile of the inventive formulation is such that a maximum steady state plasma concentration (Cₘₐₓ) of alprazolam is not higher than the maximum plasma concentration produced by the equivalent amount of alprazolam administered as an immediate release formulation TID, and a minimum steady state plasma concentration (Cₘᵢₙ) which is not lower than 75% of the minimum plasma concentration produced by the equivalent amount of alprazolam administered as an immediate release formulation TID.

In another embodiment, the pre-determined release profile provides for a bioavailability, which is equivalent to that produced by the equivalent amount of alprazolam administered as an immediate release formulation TID.

In yet another embodiment, the profile is such that the degree of fluctuation is in the range of from 50% to 100% of the degree of fluctuation produced by the equivalent amount of alprazolam administered as an immediate release formulation TID.

Formulations of the current invention are characterized by a decreased level of undesirable side effects as compared to the equivalent amount of alprazolam administered as an immediate release formulation TID. The side effects that are potentially reduced include sedation, somnolence and lightheadedness, among others.

The inventive formulations of the present invention comprise alprazolam, which may be in a micronized form, and at least one release controlling material. Due to the presence of an extended release component, the formulation provides alprazolam release extended by at least two hours beyond the release duration of XANAX XR. The novel formulation additionally provides an early and fast rise of the plasma concentration of alprazolam to provide a relief to a patient soon after administration of the drug product due to the presence of an immediate release component. In some of the embodiments, the whole amount or a part of the active agent may be present in a micronized form to improve its solubility. Preferably, at least 50% of alprazolam is in the form of particles that are less than 10 microns in size. In the most preferred embodiment, at least 90% of alprazolam is in the form of the particles that are less than 10 microns in size.

According to the invention the once a day formulation of alprazolam comprises at least one IR component and at least one XR component comprising a release controlling material. The IR component of the formulation is such that at least 90% of the active agent contained in it is released within 30 minutes. Preferably, the IR component is such that at least 90% of the active agent is released within 15 minutes. The amount of the immediate release component in the formulation is from 0.5% to 35%; preferably, from 0.5% to 20%, of the entire formulation.

The release controlling material in the formulation of the present invention is selected from ethylcellulose, polyvinyl acetate, poly (ethyl acrylate-co-methyl methacrylate-cotrimethylammonioethyl methacrylate chloride) (i.e. Eudragit® RS, Eudragit® RL), poly (ethyl acrylate-co-methyl methacrylate) (i.e. Eudragit® NE), cellulose acetate, cellulose acetate butyrate, cellulose acetate propionate, methylcellulose, waxes, and combinations thereof.

The formulations may additionally comprise a release delaying material, and optional pharmaceutically acceptable excipients, selected from wetting and solubility enhancing agents such as docusate sodium, sodium lauryl sulfate, polyethylene glycol, lecithin, poloxamer, the polysorbates, the polyoxyethylene ethers and the sorbitan esters: bulking agents such as microcrystalline cellulose, dicalcium phosphate, lactose, dextrose, mannitol, xylitol, sucrose, fructose, calcium sulfate, calcium carbonate, cellulose powder, maltodextrin, lactilol, maltilol, isomalt, talc and starch; binders such as povidone, starch, gelatin, maltodextrin, methylcellulose, hydroxypropyl cellulose, hydroxypropyl methylcellulose, hydroxyethyl cellulose, methylcellulose, polyethylene oxide, carboxymethylcellulose, calcium phosphate, dicalcium phosphate, sucrose solution, dextrose solution, dextrin, acacia, alginic acid, tragacanth and locust bean gum; disintegrants such as crosslinked sodium carboxymethylcellulose, croscarmellose, sodium starch glycolate and crospovidone, microcrystalline cellulose, methylcellulose, potassium polacrilin, low substituted hydroxypropyl cellulose, starch, soy polysaccharides; glidants such as talc, starch and colloidal silicon dioxide and the metallic stearates; lubricants selected from talc, sodium stearyl fumarate, hydrogenated vegetable oils, glyceryl palmitostearate, glyceryl behenate, poloxamer, stearic acid, stearyl alcohol, cetyl alcohol, waxes, and the metallic stearates; and buffering agents and pH modulating agents such as aluminum hydroxide, ammonium bicarbonate, ammonium carbonate, ammonium phosphate, arginine, calcium acetate, calcium ascorbate, magnesium acetate, magnesium carbonate, potassium acetate, potassium bicarbonate, potassium carbonate, potassium phosphate dibasic, potassium sodium tartrate, potassium citrate, sodium citrate, sodium phosphate monobasic, sodium phosphate dibasic, sodium phosphate tribasic, sodium acetate, sodium bicarbonate, sodium ascorbate, sodium carbonate, fumaric acid, malic acid, tartaric acid, ascorbic acid, aspartic acid, alginic acid, glutamic acid, sorbic acid, and succinic acid.

The release delaying material used in the release delaying coating may also comprise an enteric polymer, which may be selected from the group consisting of poly(methyl acrylate-co-methyl methacrylate-co-methacrylic acid)(i.e. Eudragit® FS), cellulose acetate phthalate, cellulose acetate trimelliate, poly (methacrylic acid-co-methyl methacrylate) (i.e., Eudragit® L and Eudragit® S), hydroxypropyl methylcellulose acetate succinate, hydroxypropyl methylcellulose phthalate, polyvinyl acetate phthalate, shellac and zein.

The release delaying coating typically provides a release delay of from 0.5 to 4 hours, preferably from 1 to 3 hours.

In a further embodiment of the invention, the XR component alone, or both the XR component and the IR component, is contained in at least one population of beads comprising a carrier, a binder, and the active agent, wherein each population of beads is characterized by its own rate of release. The formulations of this embodiment may be produced by a variety of methods, well known in the art. For example, release controlling material may be coated onto the alprazolam containing beads, as shown in the non-limiting Example 3 below. The release controlling material coated beads of this embodiment may be prepared by first preparing the IR beads, and then coating them with the release controlling polymer. The IR beads are prepared by liquid or dry powder drug layering onto inert substrates (cores) (e.g. cellulose spheres, silicon dioxide, starch and sugar spheres), roller compaction granulation and wet granulation with extrusion and spheronization.

The drug layering process includes the preparation of a suspension of the alprazolam in a micronized or a non-micronized form along with a binder. The binder may be hypromellose (e.g., METHOCEL™ E5 Premium LV), hydroxypropyl cellulose, hydroxyethyl cellulose, povidone, dextrin, maltodextrin, acacia, guar gum, carboxymethylcellulose, gelatin, starch, sodium alginate, sucrose solution, dextrose solution, or combinations thereof. Wetting agents may be additionally included in the formulation to assist with the dispersion of the drug in the aqueous medium. Wetting agents may include, but are not limited to, the ionic and nonionic surface active agents such as docusate sodium, sodium lauryl sulfate, polyethylene glycol, lecithin, poloxamer, the polysorbates, the polyoxyethylene ethers and the sorbitan esters. The drug suspension is applied to the core (e.g., cellulose spheres, silicon dioxide, starch or sugar spheres) in a fluid bed processor. The preferred core is sugar spheres.

The resultant drug layered bead can be overcoated with a film coating such as a hypromellose based coating (e.g., an Opadry film coating system) using a fluid bed processor to protect the bead from attrition of the drug layer during further processing, to minimize migration of the drug substance into the release controlling film during the application of the release controlling coating system and to prevent aggregation of the beads on storage.

The IR beads prepared as described above are used as substrates for the preparation of a number of populations of XR beads. The release controlling coating systems evaluated included ethylcellulose as aqueous dispersions (Surelease® Clear and Aquacoat® ECD) and in an organic solvent system, polyvinyl acetate (Kollicoat® SR 30D), Eudragit® RS 30D and Eudragit® RL 30D (poly (ethyl acrylate-co-methyl methacrylate-cotrimethylammonioethyl methacrylate chloride)) and Eudragit® NE 30 D (poly (ethyl acrylate-co-methyl methacrylate)). Additional release controlling polymers suitable for the preparation of extended release beads included cellulose acetate, cellulose acetate butyrate, cellulose acetate propionate, methylcellulose, and waxes (e.g., carnauba wax, microcrystalline wax).

To modulate the release profile of the release controlling polymer coated bead, a water soluble excipient may be added to the polymer coating system to serve as a pore former, thus increasing the permeability of the release controlling membrane. Hypromellose (METHCEL™ E5 Premium LV) is the pore former of choice for the ethylcellulose and polyvinyl acetate coating systems, whereas povidone (Kollidon K30) is the pore former of choice for the Eudragit® systems. The typical level of pore former used, expressed as a percentage of the total functional solids in the release controlling coating system, is on the range of 5% to 15%.

Other water soluble compounds can also serve as pore forming agents such as the polymers: gelatin, hydroxyethyl cellulose, hydroxypropyl cellulose, polyvinyl alcohol and Eudragit® E (poly(butyl methacrylate-co-(2-dimethylaminoethyl) methacrylate-comethyl methacrylate)), and the commercially available film coating systems such as Opadry or Opadry II products, inorganic salts (e.g., sodium and potassium chloride), organic acids and salts of organic acids.

Alternatively, the formulations of the present invention may be produced by incorporating the release controlling material into the alprazolam containing beads thus forming the XR matrix beads, as shown in Example 5. The release controlling materials useful for this application may be selected from the group consisting of ethylcellulose, hydroxypropyl cellulose, hydroxyethyl cellulose, hydroxypropyl methylcellulose, methyl cellulose, carboxymethylcellulose, acrylic acid derivatives (e.g., carbomer homopolymer type A NF and carbomer homopolymer type B NF), polyethylene oxide, carrageenan, tragacanth, xanthan gum, alginic acid (and salts thereof), polyvinyl acetate, waxes, hydrogenated vegetable oils, methacrylic acid copolymer type A NF (Eudragit® L100), methacrylic acid copolymer type B NF (Eudragit® S 100), methacrylic acid copolymer type C NF (Eudragit® L100 55), ammonio methacrylate copolymer type A NF (Eudragit® RL 100), ammonio methacrylate copolymer type B NF (Eudragit® RS 100), poly (ethyl acrylate-co-methyl methacrylate) (i.e., Eudragit® NE and Eudragit® NM); poly (methyl acrylate-co-methyl methacrylate-co-methacrylic acid) (i.e., Eudragit® FS), poly (butyl methacrylate-co-(2-dimethylaminoethyl) methacrylate-comethyl methacrylate) (i.e., Eudragit® E), Compritol 888 ATO (glyceryl behenate), Precirol ATO 5 (glyceryl palmitostearate) and PEG glyceryl esters such as Gelucire 50/13. These compounds are used in the formulation in the amount of from 5% to 50%, preferably between 10% to 40% by weight of the XR component.

The XR matrix beads may be additionally coated with a layer of the release controlling material, which may be the same or different as the one incorporated into the XR matrix beads.

The specific amount of every bead population in the formulation is determined according to the pre-determined release profile and to the rate of release characteristic for every bead population. This rate of release is influenced by such factors as the amount of the active agent, nature and amount of the release controlling material and the amount of some excipients, such as pore formers in the case of release controlling coatings.

In another embodiment of the invention, the formulation is in the form of a multilayered tablet comprising at least one layer containing the IR component, and at least one layer containing the XR component. The XR containing layer may be presented as an IR layer coated with the release controlling material. Alternatively, the XR containing layer may comprise the active agent intermixed with the release controlling material. Such a multilayered tablet may comprise several IR containing layers and/or several XR containing layers, as well as an optional release delaying coating on some of the layers, thus opening the possibility for a variety of customized release profiles.

In yet another embodiment of the invention, a once a day formulation of alprazolam is an osmotic formulation, comprising at least one osmotic core, which is a single layer core or a multiple layer core. The drug may be incorporated into one or more layers of the multiple layer core. The IR component may be present as an overcoat (e.g., hypromellose based film coat) comprising the active agent.

The total amount of alprazolam in the formulation varies between 0.25mg and 10mg.

Any form of alprazolam, such as solvates (including hydrates), pharmaceutically acceptable salts, crystalline and non-crystalline forms, optical isomers and various polymorphs, may be used in the formulations of the current invention without any limitations. In one embodiment of the invention, the alprazolam used is a crystalline polymorph characterized by a powder X-ray diffraction pattern with at least peaks at diffraction angles 2.theta. of 9.56, 19.11, and 24.22. In another embodiment of the invention, another polymorphic form characterized by a powder X-ray diffraction pattern with at least peaks at diffraction angles 2.theta. of 13.14, 18.42, and 20.18 is used.

The formulations of the present invention may be presented in a dosage form selected from a tablet, a pill, a capsule, a caplet, a troche, a sachet, a cachet, a pouch, powder or sprinkles. In one embodiment, said dosage from is selected from a capsule, a compressed tablet, sprinkles or powder that contain the formulation of the present invention in the form of at least one population of beads.

In another embodiment, the dosage form may be a multilayered tablet wherein at least one IR component is in the form of an IR layer, and at least one XR component is in the form of an IR layer coated with the release controlling material. Another embodiment of the multilayered tablet can be a tablet-in-a-tablet comprising at least one XR layer surrounded by at least one IR layer.

The following examples are illustrative of the invention, but the invention is by no means limited to these specific examples. A person of ordinary skill in the art will find in these examples but one means to implement the instant invention. Further, while the instant examples have been presented in the context of rats for experimental convenience, the methods and reagents described herein can be readily translated to human application(s) by one of ordinary skill in the art from the teachings disclosed below.

### EXAMPLES

### EXAMPLE 1. Controlled release multi-bead formulations of Alprazolam

Plasma concentration versus time curves for the alprazolam formulations containing extended release and immediate release bead populations were simulated using WinNonlin Version 5.0.1 based on pharmacokinetic data obtained from an *in vivo* study conducted in healthy subjects evaluating several alprazolam formulations and dissolution data for the separate bead populations. The dissolution data are shown in **Figure 1****.** The data are projected to a steady-state (SS) with a 24 h dosing interval for the controlled release compositions, using the linear superposition principle (WinNonlin). The extended release populations XR1, XR2 , XR3, and XR4 were selected in such a way that for in-vitro dissolution,
for XR1, 4h <=T_{60%}<=6h
for XR2, 11h <= T_{60%} <=14h
for XR3, 5.5h < T_{60%} <=7h
for XR4, 9h < T_{60%} <=11h, and at least one of the conditions selected from a following group was to be true:
1. at steady state in vivo,
   for XR1, 1.10C_{maxIR}>=C_{maxXR1}>=0.80C_{maxIR}
   for XR2, 0.90C_{maxIR}>=C_{maxXR2}>=0.60C_{maxIR}
   for XR3, 1.10C_{maxIR}>=C_{maxXR3}>=0.80C_{maxIR}
   for XR4, 0.90C_{maxIR}>=C_{maxXR4}>=0.60C_{maxIR}
2. at steady state in vivo,
   Cₘᵢₙ XR1, XR2, XR3 and XR4 is at least 75% Cₘᵢₙ IR
3. for a single initial dose in-vivo,
   for XR1, 8h <= Tₘₐₓ <=14h
   for XR2, Tₘₐₓ >= 16 h
   for XR3, 10h <= Tₘₐₓ <=18
   for XR4, Tₘₐₓ >= 16 h,
wherein C_{maxIR} and C_{minIR} refer to the concentration of drug obtained from the equivalent amount of alprazolam administered as an immediate release formulation TID.

The results of the pharmacokinetic simulation for the seven exemplary formulations are summarized in **Table 1** below. These formulations were selected as examples only, and in no way limit the range, compositions or properties of the formulations covered by the present invention.

**Table 1. Composition and Pharmacokinetic data of Multi-bead Formulations**

| | **Form-n 1** | **Form-n 2** | **Form-n 3** | **Form-n 4** | **Form-n 5** | **Form-n 6** | **Form-n 7** |
|---|---|---|---|---|---|---|---|
| % XR1 | 0 | 0 | 0 | 11 | 0 | 0 | 9 |
| % XR2 | 0 | 0 | 0 | 0 | 28 | 44 | 0 |
| % XR3 | 0 | 44 | 28 | 0 | 28 | 0 | 29 |
| % XR4 | 89 | 44 | 61 | 78 | 33 | 50 | 53 |
| %IR | 11 | 12 | 11 | 11 | 11 | 6 | 9 |
| Rel.BA (%), SS | 1.00 | 1.01 | 1.04 | 1.02 | 1.03 | 0.98 | 0.99 |
| Deg of fluctuation, SS | 16.9 | 22.6 | 20.3 | 20.2 | 21.8 | 16.8 | 22.5 |

The dissolution profiles of controlled release multi-bead formulations are presented in **Table 2.**

**Table 2. Dissolution profiles for controlled release formulations**

| | Form-n 1 | Form-n 2 | Form-n 3 | Form-n 4 | Form-n 5 | Form-n 6 | Form-n 7 |
|---|---|---|---|---|---|---|---|
| t₂₅ | 3.5 | 3 | 3.2 | 3 | 2 | 3.5 | 2.5 |
| t₅₀ | 8 | 6 | 6.5 | 7 | 5.5 | 8 | 5.5 |
| t₇₅ | 12 | 10 | 10.5 | 12 | 11 | 14 | 10 |
| t₈₀ | 14 | 11 | 12 | 14 | 13 | 16 | 12 |

The extended release populations XR1-XR4 may be prepared starting from the IR beads by methods represented in the non-limiting Examples 2-4 below. Thus, the XR1 population is exemplified by beads B3 or B5 (Example 4); XR2 population is exemplified by beads B1, B2 or B7 (Example 3); XR3 population is exemplified by beads B4 or B6 ( Example 4), and XR4 population is exemplified by beads B8 (Example 4).

### EXAMPLE 2. Immediate release beads preparation

A suspension of the micronized alprazolam and hypromellose (e.g., METHOCEL™ E5 Premium LV) binder was prepared. The drug suspension was applied to Sugar Spheres, NF cores in a fluid bed processor. The resultant drug containing beads were overcoated with a film of an Opadry film coating system using a fluid bed processor.

Examples of three immediate release bead formulations are provided in **Table 3.**

**Table 3. Alprazolam IR beads - Drug Load 0.5% w/w, 1% w/w and 2.0% w/w**

| | **IRLP - 0.5% w/w** | **IRHP - 1.0% w/w** | **IRHP - 2.0% w/w** |
|---|---|---|---|
| **Strength (label claim) % (w/w)** | 0.5 | 1.0 | 2.0 |
| **Component^{a}** | **Quantity (g)** | **Quantity (g)** | **Quantity (g)** |
| Alprazolam, USP (micronized) | 10.91 | 120.00 | 240.00 |
| Sugar Spheres, NF (30/35 mesh) | 2012.5 | 10932 | 10812 |
| Hypromellose (Type 2910), USP (METHOCEL™ E5 Premium LV) | 41.56 | 227.64 | 227.64 |
| Opadry II White (33G28523) | 132 | 720.00 | 720 |
| Sterile Water for Irrigation, USP^{b} | 1245.1 | 7498.4 | 9092.6 |

| | | | |
|---|---|---|---|
| a. The drug layering dispersion and film coat formulations are prepared at a 20% overage in order to apply the target quantity per batch. b. Removed during processing | | | |

### EXAMPLE 3. Extended release beads preparation: coated bead

A series of extended release bead formulations were developed. The IR beads of Example 2 served as a substrate for the extended release bead preparation. The IR beads were coated with release controlling coating systems such as Eudragit® NE 30 D (poly (ethyl acrylate-co-methyl methacrylate)).

To modulate the release profile of the extended release (XR) beads, a water soluble excipient was added to the polymer coating system to serve as a pore former thus increasing the permeability of the extended release membrane. Povidone (Kollidon K30) was the pore former of choice for the Eudragit® system.

Examples of two extended release (XR) bead formulations (B1 and B2) are provided in **Table 4.**

**Table 4. Representative Alprazolam Extended Release Beads - Drug load 1.72% w/w and 1.68% w/w**

| | **B1** | **B2** |
|---|---|---|
| **Strength (label claim) % (w/w)** | 1.72 | 1.68 |

| **Component^{a}** | **Quantity (g)** | **Quantity (g)** |
|---|---|---|
| IRHP Pellets (2.0% w/w) | 1894.3 | 1851.0 |
| Eudragit® NE 30 D^{b} | 414.63 | 482.88 |
| Povidone, USP (K 30) (Kollidon 30) | 13.82 | 16.10 |
| Talc, USP | 167.53 | 188.00 |
| Sterile Water for Irrigation, USP^{c} | 760.15 | 885.28 |

| | | |
|---|---|---|
| a. The extended release coating formulations is prepared at a 20% overage in order to apply the target quantity per batch. b. The usage level of Eudragit® NE 30 D is based on a solids assay value of 30.4% w/w. c. Removed during processing | | |

### EXAMPLE 4. Extended release beads with the release delaying coating

Extended release beads with the release delaying coating were produced by coating the extended release beads prepared by the process of Example 3 with Eudragit® FS 30 D (poly(methyl acrylate-co-methyl methacrylate-co-methacrylic acid)) as an enteric coating system.

Representative formulation of such beads, B4, is provided in **Table 6** on the base of the B3 beads **(Table 5).**

**Table 5. Alprazolam Extended Release B3 Beads - Drug load 0.4 w/w**

| | B3 Beads |
|---|---|
| Strength (label claim) % (w/w) | 0.4 |

| Component^{a} | Quantity (g) |
|---|---|
| IRLP Pellets (0.5% w/w) | 1780.9 |
| Eudragit® NE 30 D^{b} | 596.63 |
| Povidone, USP (K 30) (Kollidon 30) | 19.79 |
| Talc, USP | 212.23 |
| Sterile Water for Irrigation, USP^{c} | 1088.3 |

| | |
|---|---|
| a. The extended release coating formulations is prepared at a 20% overage in order to apply the target quantity per batch. b. The usage level of Eudragit® NE 30 D is based on a solids assay value of 30.4% w/w. c. Removed during processing | |

**Table 6. Alprazolam Extended Release B4 Beads with the delayed release coating - Drug load 0.32 w/w**

| | B4 beads |
|---|---|
| Strength (label claim) % (w/w) | 0.32 |

| Component^{a} | Quantity (g) |
|---|---|
| XR3 Beads (0.4% w/w) | 1754.4 |
| Eudragit® FS 30 D^{b} | 1032.0 |
| Triethyl Citrate, NF | 15.48 |
| Talc, USP | 120.54 |
| Sterile Water for Irrigation, USP^{c} | 887.51 |

| | |
|---|---|
| a. The enteric coating formulation is prepared at a 20% overage in order to apply the target quantity per batch. b. The usage level of Eudragit® FS 30 D is based on a solids assay value of 30.3% w/w. c. Removed during processing | |

Further examples of the extended release beads with the release delaying coating (B6 and B8) are presented in Table 8 on the base of the extended release beads (B5 and B7) (Table 7).

**Table 7. B5 - 0.84% w/w and B7 -1.68% w/w Pellet Formulations**

| | B5 | B7 |
|---|---|---|
| **Strength (label claim) % (w/w)** | 0.84 | 1.68 |

| **Component^{a}** | **Quantity (g)** | **Quantity (g)** |
|---|---|---|
| IRHP -1.0% w/w | 4856.6 | NA |
| IRHP -2.0% w/w | NA | 4880.0 |
| Eudragit® NE 30 D^{b} | 1310.1 | 1273.1 |
| Povidone, USP (K 30) (Kollidon 30) | 43.67 | 42.46 |
| Talc, USP | 506.75 | 495.66 |
| Sterile Water for Irrigation, USP^{c} | 600.50 | 885.28 |

| | | |
|---|---|---|
| NA = Not Applicable a. The extended release coating formulations is prepared at a 20% overage in order to apply the target quantity per batch. b. The usage level of Eudragit® NE 30 D is based on a solids assay value of 30% w/w. c. Removed during processing | | |

**Table 8. B6 - 0.67% w/w and B8 - 1.34% w/w Pellet Formulations**

| | **B6** - **0.67% w/w** | **B8 - 1.34% w/w** |
|---|---|---|
| **Strength (label claim) % (w/w)** | 0.67 | 1.34 |

| **Component**^{a} | **Quantity (g)** | **Quantity (g)** |
|---|---|---|
| XR5 - 0.84% w/w | 4777.9 | NA |
| XR7 -1.34% w/w | NA | 4777.9 |
| Eudragit® FS 30 D^{b} | 2838.5 | 2838.5 |
| Triethyl Citrate, NF | 42.14 | 42.14 |
| Talc, USP | 328.4 | 328.4 |
| Sterile Water for Irrigation, USP ^{c} | 2459.1 | 2459.1 |

| | | |
|---|---|---|
| NA = Not Applicable a. The enteric coating formulation is prepared at a 20% overage in order to apply the target quantity per batch. b. The usage level of Eudragit® FS 30 D is based on a solids assay value of 30% w/w. c. Removed during processing | | |

### EXAMPLE 5. Extended release beads preparation: matrix bead

The extended release matrix bead is manufactured by the process of roller compaction or extrusion/spheronization. An example matrix bead formulation for extrusion/spheronization is provided in **Table 9.**

**Table 9. Alprazolam Matrix Beads - Drug load 1.70 w/w**

| | Extended release matrix beads |
|---|---|
| Strength (label claim) % (w/w) | 1.70 |
| Component^{a} | Quantity (g) |
| Alprazolam, USP (micronized) | 17.0 |
| Ammonio Methacrylate Copolymer Type B Ph. Eur. | 300.0 |
| Microcrystalline Cellulose, NF (Avicel PH 101) | 450.0 |
| Maltodextrin, NF (Maltrin M150) | 198.0 |
| Talc, USP | 20.0 |
| Povidone, USP (Kollidon K30) | 15.0 |
| Sterile Water for Irrigation, USP ^{c} | Qs |

### EXAMPLE 6. Extended Release/Immediate Release (XR/IR) bilayered tablet formulation

A bilayer Extended Release/Immediate Release (XR/IR) tablet is manufactured by the process comprising the following steps:
1. Immediate Release Granulation:
   Bulk excipients, such as maltodextrin, microcrystalline cellulose, pregelatinized starch and croscarmellose, are first screened through a 40 mesh sieve and then dry blended in a high shear granulator (Glatt-Powrex vertical granulator). The povidone is dispersed and dissolved in a sufficient quantity of water to granulate the mixture of the dry excipients. The micronized alprazolam is then added to the povidone solution and dispersed. The resulting dispersion is added to the dry excipients while granulating in the high shear granulator. The resulting granulation is dried in an oven or fluid bed dryer (Glatt GPCG-1) and then screened to obtain an optimal particle size distribution. The granules are then blended with colloidal silicon dioxide, talc and magnesium stearate in a suitable blender (e.g. a twin shell - Patterson Kelly blender).
2. Extended Release Granulation
   Similarly, bulk excipients are first screened through a 40 mesh sieve. Polyethylene oxide, methacrylic acid copolymer Type A, maltodextrin and microcrystalline cellulose, are dry blended in a high shear granulator (Glatt-Powrex vertical granulator). The povidone is dispersed and dissolved in a sufficient quantity of a mixture of water and alcohol to granulate the mixture of the dry excipients. The micronized alprazolam is then added to the povidone solution and dispersed. The resulting dispersion is added to the dry excipients while granulating in the high shear granulator. The resulting granulation is dried in an oven or fluid bed dryer (Glatt GPCG-1) and then screened to obtain an optimal particle size distribution. The granules are then blended with the colloidal silicon dioxide, talc and magnesium stearate in a suitable blender (e.g. a twin shell - Patterson Kelly blender).
3. Bilayer Tablet Compression
   The Immediate Release and Extended Release granulations are simultaneously compressed into a bilayer tablet using a bilayer rotary tablet press (Kilian S250 ZS) and appropriate tooling.

The composition of the bilayered tablet is presented in Table 10.

**Table 10. Extended Release/Immediate Release (XR/IR) bilayer tablet formulation**

| **Immediate Release Layer** | | |
|---|---|---|
| Component | Usage (%) | Usage (gm) |
| Alprazolam, USP (micronized) | 0.2 | 2 |
| Maltodextrin, NF (Maltrin M150) | 20 | 200 |
| Microcrystalline Cellulose, NF (Avicel PH 101) | 42 | 420 |
| Pregelatinized Starch, NF | 15 | 150 |
| Croscarmellose Sodium, NF (Ac-Di-Sol) | 15 | 150 |
| Povidone, USP (Kollidon K30) | 5 | 50 |
| Talc, USP | 1 | 10 |
| Colloidal Silicon Dioxide, NF (CAB-O-SIL M5P) | 0.8 | 8 |
| Magnesium Stearate | 1 | 10 |
| Purified Water, USP* | NA | Qs |
| Total | 100 | 1000 |
| * removed during processing | | |

| **Extended Release Layer** | Usage (%) | Usage (gm) |
|---|---|---|
| Alprazolam, USP (micronized) | 0.8 | 8 |
| Polyethylene Oxide, NF (Polyox WSR 301) | 25 | 250 |
| Methacrylic Acid Copolymer Type C,NF(Eudragit L100-55) | 25 | 250 |
| Maltodextrin, NF (Maltrin M150) | 20 | 200 |
| Microcrystalline Cellulose, NF (Avicel PH 101) | 21.2 | 212 |
| Povidone, USP (Kollidon K30) | 5 | 50 |
| Talc, USP | 1 | 10 |
| Colloidal Silicon Dioxide, NF (CAB-O-SIL M5P) | 1 | 10 |
| Magnesium Stearate | 1 | 10 |
| Alcohol, USP* | NA | Qs |
| Purified Water, USP* | NA | Qs |
| Total | 100 | 1000 |
| * removed during processing | | |

While the invention has been described in connection with specific embodiments thereof, it will be understood that it is capable of further modifications and this application is intended to cover any variations, uses, or alterations of the invention following. In general, the principles of the invention and including such departures from the present disclosure as come within known or customary practice within the art to which the invention pertains and as may be applied to the essential features hereinbefore set forth and as follows in the scope of the appended claims.

## Claims

1. A formulation of alprazolam for once a day oral administration to a mammalian subject, comprising alprazolam as an active agent, which is released from the formulation along a pre-determined release profile, said formulation comprising at least one population of immediate release alprazolam-containing beads (IR), and at least one population of extended release (XR) alprazolam-containing beads comprising a release controlling material, wherein at least 90% of the active agent contained in the IR component is released in vivo within 30 minutes, wherein the release controlling material contained in the XR component is selected from a group consisting of ethylcellulose, polyvinyl acetate, poly (ethyl acrylate-co-methyl methacrylate-cotrimethylammonioethyl methacrylate chloride), poly (ethyl acrylate-co-methyl methacrylate), cellulose acetate, cellulose acetate butyrate, cellulose acetate propionate, methylcellulose, and waxes.

2. The formulation of claim 1, wherein the beads include, without any limitations on the nature and size thereof, any particles, spheres, granules, pellets, particulates or any structural units that may be incorporated into an oral dosage form.

3. The formulation of claim 1 wherein at least one of the immediate release (IR) components or extended release (XR) components additionally comprises a release delaying coating.

4. The formulation of claim 3, wherein said release delaying coating comprises an enteric polymer selected from a group consisting of poly(methyl acrylate-co-methyl methacrylate-co-methacrylic acid), cellulose acetate phthalate, cellulose acetate trimellitate, (poly(methacrylic acid-co-methyl methacrylate)), hydroxypropyl methylcellulose acetate succinate, hydroxypropyl methylcellulose phthalate, polyvinyl acetate phthalate, shellac and zein.

5. The formulation of claim 1, wherein said formulation provides for a maximum steady state plasma concentration (Cₘₐₓ) of alprazolam which is not higher than the maximum plasma concentration produced by the equivalent amount of alprazolam administered as an immediate release formulation TID, and a minimum steady state plasma concentration (Cₘᵢₙ) which is not lower than 75% of the minimum plasma concentration produced by the equivalent amount of alprazolam administered as an immediate release formulation TID.

6. The formulation of claim 1, further comprising at least one excipient selected from the group consisting of wetting agents, solubility enhancing agents, disintegrants, binders, lubricants, antitacking agents, and glidants.

7. The formulation of claim 1 wherein at least 50% of alprazolam is in the form of particles that are less than 10 microns in size.

8. The formulation of claim 1, wherein at least 90% of alprazolam is in the form of particles that are less than 10 microns in size.

9. The formulation of claim 1 wherein at least said extended release component is contained in at least one population of beads comprising a carrier, a binder, and the active agent, and wherein each population of beads is **characterized by** its own rate of release.

10. The formulation of claim 9 wherein said release controlling material is incorporated into the alprazolam containing beads; coated onto the alprazolam containing beads; or both.

11. The formulation of claim 9, wherein a specific amount of every bead population is determined according to the pre-determined release profile.

12. The formulation of claim 1, wherein the active ingredient is selected from a group consisting of alprazolam, solvates of alprazolam, pharmaceutically acceptable salts of alprazolam, crystalline and non-crystalline forms of alprazolam, optical isomers of alprazolam, and polymorphs of alprazolam.

13. The formulation of claim 12 wherein alprazolam is a crystalline polymorph that comprises either a powder X-ray diffraction pattern with at least peaks at diffraction angles 2.theta. of 9.56, 19.11, and 24.22, or a powder X-ray diffraction pattern with at least peaks at diffraction angles 2.theta. of 13.14, 18.42, and 20.18.

14. The formulation of claim 1, wherein the total amount of alprazolam in the formulation is from 0.25 to 10 mg.

15. The formulation of claim 14, wherein the amount of the immediate release component is from 0.5% to 35% of the total amount of the active agent in the formulation.

16. The formulation of claim 1 in a dosage form selected from a tablet, a pill, a capsule, a caplet, a troche, a sachet, a cachet, a pouch, or sprinkles.

17. The formulation of claim 16, wherein said dosage form is a multilayered tablet such that at least one immediate release (IR) component is in a form of an immediate release (IR) layer, and at least one extended release (XR) component is in the form of immediate release (IR) layer coated with the release controlling material.

18. The formulation of claim 16, wherein said dosage form is a multilayered tablet such that at least one IR component is in a form of an IR layer, and at least one XR component is in a form of a layer comprising the active agent intermixed with the release controlling material.

19. The formulation of claim 1 for the use in the treatment of a pathological condition in a mammalian subject.

20. The formulation of claim 19 for use in the treatment of generalized anxiety disorder, panic disorder, and anxiety associated with other CNS disorders.

21. The formulation of claim 19, which produces an improved side effect profile as compared to that produced by the equivalent amount of alprazolam administered as an immediate release formulation TID.

## Patentansprüche

1. Alprazolam-Zubereitung zur einmaligen täglichen Verabreichung an einen Säuger, umfassend Alprazolam als Wirkstoff, der aus der Zubereitung nach einem vorbestimmten Freisetzungsprofil freigesetzt wird, wobei die Zubereitung mindestens eine Population von Alprazolam enthaltenden Perlen mit sofortiger Freisetzung (IR) und mindestens eine Population von Alprazolam enthaltenden Perlen mit verlängerter Freisetzung (XR), die ein die Freisetzung steuerndes Material enthalten, umfasst, wobei mindestens 90 % des in der IR-Komponente enthaltenen Wirkstoffs in vivo innerhalb von 30 Minuten freigesetzt werden, wobei das in der XR-Komponente enthaltene, die Freisetzung steuernde Material aus einer Gruppe ausgewählt ist, die besteht aus Ethylcellulose, Polyvinylacetat, Poly-(ethylacrylat-co-methylmethacrylat-co-trimethylammmonioethyl-methacrylat-chlorid), Poly-(ethylacrylat-co-methylmethacrylat), Celluloseacetat, Celluloseacetobutyrat, Celluloseacetopropionat, Methylcellulose und Wachsen.

2. Zubereitung nach Anspruch 1, wobei die Perlen, ohne Beschränkung in Bezug auf ihre Art und Größe, beliebige Teilchen, Kügelchen, Granalien, Pellets, Partikel oder beliebige strukturelle Einheiten, die einer oralen Dosierungsform einverleibt werden können, umfassen.

3. Zubereitung nach Anspruch 1, wobei mindestens eine der Komponenten mit sofortiger Freisetzung (IR) oder der Komponenten mit verlängerter Freisetzung (XR) zusätzlich einen die Freisetzung verzögernden Überzug umfasst.

4. Zubereitung nach Anspruch 3, wobei der die Freisetzung verzögernde Überzug ein erst im Darm lösliches Polymer umfasst, das aus einer Gruppe ausgewählt ist, die besteht aus Poly-(methylacrylat-co-methylmethacrylat-co-methacrylsäure), Celluloseacetatphthalat, Celluloseacetotrimellitat, Poly-(methacrylsäure-co-methylmethacrylat), Hydroxypropylmethylcelluloseacetatsuccinat, Hydroxypropylmethylcellulose-phthalat, Polyvinylacetatphthalat, Schellack und Zein.

5. Zubereitung nach Anspruch 1, wobei die Zubereitung eine maximale Stationärzustand-Plasmakonzentration (Cₘₐₓ) von Alprazolam, die nicht höher als die maximale Plasmakonzentration ist, die durch eine äquivalente Menge an Alprazolam, die als Zubereitung mit sofortiger Freisetzung t.i.d. verabreicht wird, erzeugt wird, und eine minimale Stationärzustand-Plasmakonzentration (Cₘᵢₙ), die nicht unter 75 % der minimalen Plasmakonzentration ist, die durch eine äquivalente Menge an Alprazolam, die als Zubereitung mit sofortiger Freisetzung t.i.d. verabreicht wird, erzeugt wird, bereitstellt.

6. Zubereitung nach Anspruch 1, ferner umfassend mindestens ein Exzipiens, das aus einer Gruppe ausgewählt ist, die besteht aus Netzmitteln, Mitteln zur Verbesserung der Löslichkeit, Sprengmitteln, Bindemitteln, Schmiermitteln, Mitteln zur Verhinderung der Klebrigkeit und Gleitmitteln.

7. Zubereitung nach Anspruch 1, wobei mindestens 50 % des Alprazolams in Form von Teilchen mit einer Größe von weniger als 10 µm vorliegen.

8. Zubereitung nach Anspruch 1, wobei mindestens 90 % des Alprazolams in Form von Teilchen mit einer Größe von weniger als 10 µm vorliegen.

9. Zubereitung nach Anspruch 1, wobei mindestens die Komponente mit verlängerter Freisetzung in mindestens einer Population von Perlen, die einen Träger, ein Bindemittel und den Wirkstoff umfassen, enthalten ist, und wobei jede Population von Perlen durch eine eigene Freisetzungsgeschwindigkeit gekennzeichnet ist.

10. Zubereitung nach Anspruch 9, wobei das die Freisetzung steuernde Material den Alprazolam enthaltenden Perlen einverleibt ist und/oder auf die Alprazolam enthaltenden Perlen aufgebracht ist.

11. Zubereitung nach Anspruch 9, wobei eine bestimmte Menge der einzelnen Perlenpopulationen entsprechend dem vorbestimmten Freisetzungsprofil festgelegt wird.

12. Zubereitung nach Anspruch 1, wobei der Wirkstoff aus einer Gruppe ausgewählt ist, die besteht aus Alprazolam, Solvaten von Alprazolam, pharmazeutisch verträglichen Salzen von Alprazolam, kristallinen und nicht kristallinen Formen von Alprazolam, optischen Isomeren von Alprazolam und Polymorphen von Alprazolam.

13. Zubereitung nach Anspruch 12, wobei es sich beim Alprazolam um ein kristallines Polymorphes handelt, das entweder ein Pulver-Röntgenbeugungsmuster, das mindestens Peaks bei Beugungswinkeln 2 theta von 9,56, 19,11 und 24,22 aufweist, oder ein Pulver-Röntgenbeugungsmuster, das mindestens Peaks bei Beugungswinkeln 2 theta von 13,14, 18,42 und 20,18 aufweist, umfasst.

14. Zubereitung nach Anspruch 1, wobei die Gesamtmenge von Alprazolam in der Zubereitung 0,25 bis 10 mg beträgt.

15. Zubereitung nach Anspruch 14, wobei die Menge der Komponente mit sofortiger Freisetzung 0,5 % bis 35 % der Gesamtmenge des Wirkstoffs in der Zubereitung beträgt.

16. Zubereitung nach Anspruch 1 in einer Dosierungsform, die ausgewählt ist aus einer Tablette, einer Pille, einer Kapsel, eines Caplets, einer Pastille, eines Tütchens, eines Cachets, eines Beutels oder Streuseln.

17. Zubereitung nach Anspruch 16, wobei es sich bei der Dosierungsform um eine mehrlagige Tablette handelt, so dass mindestens eine Komponente mit sofortiger Freisetzung (IR) in Form einer Schicht mit sofortiger Freisetzung (IR) vorliegt und mindestens eine Komponente mit verlängerter Freisetzung (XR) in Form einer Schicht mit sofortiger Freisetzung (IR), die mit dem die Freisetzung steuernden Material überzogen ist, vorliegt.

18. Zubereitung nach Anspruch 16, wobei es sich bei der Dosierungsform um eine mehrlagige Tablette handelt, so dass mindestens eine IR-Komponente in Form einer IR-Schicht vorliegt und mindestens eine XR-Komponente in Form einer Schicht vorliegt, die den Wirkstoff im Gemisch mit dem die Freisetzung steuernden Material umfasst.

19. Zubereitung nach Anspruch 1 zur Verwendung bei der Behandlung eines pathologischen Zustands bei einem Säuger.

20. Zubereitung nach Anspruch 19 zur Verwendung bei der Behandlung von generalisierten Angststörungen, panischen Störungen und Angst in Verbindung mit anderen ZNS-Störungen.

21. Zubereitung nach Anspruch 19, die ein verbessertes Nebenwirkungsprofil ergibt, verglichen mit dem Profil, das sich durch eine äquivalente Menge an Alprazolam, das t.i.d. als Zubereitung mit sofortiger Freisetzung verabreicht wird.

## Revendications

1. Formulation d'alprazolam pour une administration par voie orale une fois par jour à un sujet mammifère, comprenant de l'alprazolam à titre d'agent actif, qui est libéré de la formulation selon un profil de libération prédéterminé, ladite formulation comprenant au moins une population de perles contenant de l'alprazolam à libération immédiate (IR) et au moins une population de perles contenant de l'alprazolam à libération prolongée (XR) comprenant un matériau de contrôle de libération, dans laquelle au moins 90 % de l'agent actif contenu dans le composant IR sont libérés in vivo en l'espace de 30 minutes, dans laquelle le matériau de contrôle de libération contenu dans le composant XR est choisi dans le groupe constitué par l'éthylcellulose, l'acétate de polyvinyle, le copolymère d'acrylate d'éthyle/méthacrylate de méthyle/chlorure de méthacrylate de méthylammonioéthyle, le copolymère d'acrylate d'éthyle/méthacrylate de méthyle, l'acétate de cellulose, l'acétobutyrate de cellulose, l'acétopropionate de cellulose, la méthylcellulose, et les cires.

2. Formulation selon la revendication 1, dans laquelle les perles comprennent, sans aucune limitation concernant leur nature et leur taille, n'importe quels particules, sphères, granules, pastilles, particules ou n'importe quelles unités structurelles, qui peuvent être incorporés dans une forme posologique à usage oral.

3. Formulation selon la revendication 1, dans laquelle au moins l'un des composants à libération immédiate (IR) ou des composants à libération prolongée (XR) comprend de plus un enrobage retardant la libération.

4. Formulation selon la revendication 3, dans laquelle ledit enrobage retardant la libération comprend un polymère entérique choisi dans le groupe constitué par le copolymère d'acrylate de méthyle/méthacrylate de méthyle/acide méthacrylique, l'acétophtalate de cellulose, l'acétotrimellitate de cellulose, le copolymère d'acide méthacrylique/méthacrylate de méthyle, l'acétosuccinate d'hydroxypropylméthylcellulose, le phtalate d'hydroxypropylméthylcellulose, le poly(acétophtalate de vinyle), la gomme laque et la zéine.

5. Formulation selon la revendication 1, laquelle formulation réalise une concentration plasmatique stable maximale (Cₘₐₓ) d'alprazolam qui n'est pas supérieure à la concentration plasmatique maximale produite par une quantité équivalente d'alprazolam administrée sous la forme d'une formulation à libération immédiate trois fois par jour, et une concentration plasmatique stable minimale (Cₘᵢₙ) qui n'est pas inférieure à 75 % de la concentration plasmatique minimale produite par une quantité équivalente d'alprazolam administrée sous la forme d'une formulation à libération immédiate trois fois par jour.

6. Formulation selon la revendication 1, comprenant en outre au moins un excipient choisi dans l'ensemble constitué par les agents mouillants, les agents amplifiant la solubilité, les agents délitants, les liants, les lubrifiants, les agents anti-collants, et les agents de glissement.

7. Formulation selon la revendication 1, dans laquelle au moins 50 % d'alprazolam sont sous la forme de particules ayant une taille inférieure à 10 micromètres.

8. Formulation selon la revendication 1, dans laquelle au moins 90 % d'alprazolam sont sous la forme de particules ayant une taille inférieure à 10 micromètres.

9. Formulation selon la revendication 1, dans laquelle au moins ledit composant à libération prolongée est contenu dans au moins une population de perles comprenant un support, un liant, et l'agent actif, et dans laquelle chaque population de perles est **caractérisée par** sa propre vitesse de libération.

10. Formulation selon la revendication 9, dans laquelle ledit matériau de contrôle de libération est incorporé dans les perles contenant de l'alprazolam ; déposé sous forme d'enrobage sur les perles contenant de l'alprazolam ; ou les deux.

11. Formulation selon la revendication 9, dans laquelle une quantité spécifique de chaque population de perles est déterminée conformément au profil de libération prédéterminé.

12. Formulation selon la revendication 1, dans laquelle l'agent actif est choisi dans l'ensemble constitué par l'alprazolam, les solvates d'alprazolam, les sels pharmaceutiquement acceptables d'alprazolam, les formes cristallines et non cristallines d'alprazolam, les isomères optiques d'alprazolam, et les polymorphes d'alprazolam.

13. Formulation selon la revendication 12, dans laquelle l'alprazolam est un polymorphe cristallin qui comprend soit un motif de diffraction des rayons X par la technique des poudres ayant au moins des pics aux angles de diffraction 2•thêta de 9,56, 19,11 et 24,22, soit un motif de diffraction des rayons X par la technique des poudres ayant au moins des pics aux angles de diffraction 2•thêta de 13,14, 18,42 et 20,18.

14. Formulation selon la revendication 1, dans laquelle la quantité totale d'alprazolam dans la formulation est de 0,25 à 10 mg.

15. Formulation selon la revendication 14, dans laquelle la quantité du composant à libération immédiate est de 0,5 % à 35 % de la quantité totale de l'agent actif dans la formulation.

16. Formulation selon la revendication 1, sous une forme posologique choisie parmi un comprimé, une pilule, une capsule, un caplet, une pastille, un sachet, un cachet, une poche, ou des vermicelles.

17. Formulation selon la revendication 16, dans laquelle ladite forme posologique est un comprimé multicouche tel qu'au moins un composant à libération immédiate (IR) soit sous la forme d'une couche à libération immédiate (IR), et au moins un composant à libération prolongée (XR) soit sous la forme d'une couche à libération immédiate (IR) enrobée du matériau de contrôle de libération.

18. Formulation selon la revendication 16, dans laquelle ladite forme posologique est un comprimé multicouche tel qu'au moins un composant IR soit sous la forme d'une couche IR, et au moins un composant XR soit sous la forme d'une couche comprenant l'agent actif mélangé avec le matériau de contrôle de libération.

19. Formulation selon la revendication 1, pour une utilisation dans le traitement d'un état pathologique chez un sujet mammifère.

20. Formulation selon la revendication 19, pour une utilisation dans le traitement d'un trouble de l'anxiété généralisé, d'un trouble panique, et d'une anxiété associée à d'autres troubles du SNC.

21. Formulation selon la revendication 19, qui produit un meilleur profil d'effets secondaires en comparaison avec celui produit par une quantité équivalente d'alprazolam administrée sous la forme d'une formulation à libération immédiate trois fois par jour.
